# EUROPÄISCHE PATENTSCHRIFT

(11) **EP 0 322 348 B1**
(45) Veröffentlichungstag und Bekanntmachung des Hinweises auf die Patenterteilung: **02.02.1994**
(21) Anmeldenummer: 88710054.3
(22) Anmeldetag: 16.12.1988
(51) Int. Cl.: C07K 1/04, C07K 7/06

(54) **Säurelabile Ankergruppen zur Synthese von Peptidamiden mittels Festphasenmethode**
Acidically non-stable anchoring groups for solid-phase peptide amide synthesis
Groupes d'ancrage labiles envers les acides pour la synthèse des peptides amidés en phase solide

(30) Priorität: 22.12.1987 DE 3743620; 01.06.1988 DE 3818576
(43) Veröffentlichungstag der Anmeldung: 28.06.1989
(73) Patentinhaber: HOECHST AKTIENGESELLSCHAFT, 65926 Frankfurt am Main (DE)
(72) Erfinder: Breipohl, Gerhard, Dr., D-6000 Frankfurt am Main 71 (DE); Knolle, Jochen, Dr., D-6239 Kriftel (DE); Stüber, Werner, Dr., D-3551 Lahntal (DE)

(56) Entgegenhaltungen:
- EP-A- 0 287 882
- CHEMICAL ABSTRACTS, Band 76, Nr. 1, 3. Januar 1972, Seite 369, Zusammenfassung Nr. 4166a, Columbus, Ohio, US; & GB-A-1 238 415 (FARBWERKE HOECHST A.-G.) 07-07-1971

## Beschreibung

Die Erfindung betrifft neue Spacer und ihre Herstellungsverfahren sowie die Synthese von Peptidamiden mittels Festphasenmethode unter Verwendung dieser säurelabilen Ankergruppen.

Zur Herstellung von Peptidamiden mittels Festphasensynthese verwendet man im allgemeinen Benzhydrylamin- oder Methylbenzhydrylaminharze, wie sie z.B. bei J.P. Tam et al., Tetrahedron Lett. 22, 2851 (1981) beschrieben sind. Eine weitere Methode besteht in der Ammonolyse von trägergebundenen Peptidbenzylestern (C. Ressler et al., J. Am. Chem. Soc. 76, 3107 (1954)). Beide Methoden sind charakterisiert durch die zur Abspaltung des "Spacers" notwendige starke Säure (flüssiger Fluorwasserstoff oder Trifluormethansulfonsäure), Nebenreaktionen oder unvollständige Abspaltung.

Der Erfindung liegt daher die Aufgabe zugrunde, neue Spacer zu finden, die eine mildere und bessere Abspaltung von Peptidamiden vom Trägerharz ermöglichen.

Diese Aufgabe wird erfindungsgemäß gelöst durch die Verbindungen der allgemeinen Formel I 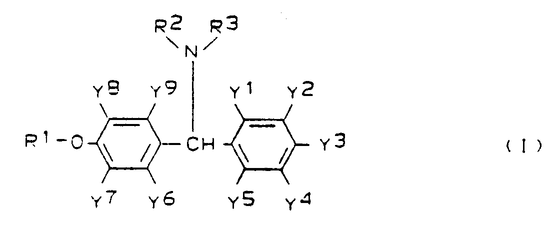
worin
- R¹: (C₁-C₈)-Alkyl,
- R²: eine schwach sauer oder basisch abspaltbare Aminoschutzgruppe,
- R³: Wasserstoff oder (C₁-C₄)-Alkyl,
- Y¹, Y², Y³, Y⁴, Y⁵, Y⁶, Y⁷, Y⁸, Y⁹: Wasserstoff, (C₁-C₄)-Alkyl, (C₁-C₄)-Alkoxy oder -O-(CH₂)ₙ-COOH, wobei die Reste gleich oder verschieden sein können, ein Rest aber -O-(CH₂)ₙ-COOH ist, oder
- Y¹, Y², Y⁵, Y⁶, Y⁷, Y⁸, Y⁹: Wasserstoff, (C₁-C₄)-Alkyl oder (C₁-C₄)-Alkoxy, wobei die Reste gleich oder verschieden sein können, Y³ (C₁-C₈)-Alkoxy oder Wasserstoff und Y⁴ -(CH₂)ₙ-COOH oder -NH-CO-(CH₂)ₙ-COOH, und
- n: eine ganze Zahl von 1 bis 6
bedeuten.

Bevorzugt sind Verbindungen der allgemeinen Formel I worin R¹ Methyl und n eine ganze Zahl von 1, 2 oder 3 sind.

Ebenfalls bevorzugt sind Verbindungen dieser allgemeinen Formel I, worin oder eine Urethanschutzgruppe-insbesondere Fmoc-und R³ Wasserstoff bedeutet.

Ferner stehen die Reste Y¹-Y⁹ insbesondere für Methyl oder Methoxy, wobei jedoch ein Rest -O-(CH₂)ₙ-COOH und mindestens 4 dieser Reste Wasserstoff sind oder die Reste Y¹, Y², Y⁵-Y⁹ für Methyl oder Methoxy, wobei jedoch mindestens 4 dieser Reste Wasserstoff sind, Y³ für Methyl und Y⁴ für -(CH₂)ₙ-COOH.
Vorzugsweise steht ein Rest Y¹, Y³, Y⁵, Y⁷ oder Y⁸ für -O-(CH₂)ₙ-COOH.

In Verbindungen, in denen Y⁴ -(CH₂)ₙ-COOH bedeutet, ist Y³ (C₁-C₄)-Alkoxy, insbesondere Methoxy und n = 2; falls Y⁴ für -NH-CO-(CH₂)ₙ-COOH steht, sind bevorzugt Y¹ und Y³ Methoxy und n = 2.

Alkyl und Alkoxy können geradkettig oder verzweigt sein.

R² steht für eine basenlabile oder gegen schwache Säuren labile Schutzgruppe, wie z.B. Urethanschutzgruppen, wie Fmoc, Ddz, Bpoc, Msc, Peoc, Pse und Tse, vorzugsweise Fmoc.

Geeignete Schutzgruppen sind bei Hubbuch, Kontakte (Merck) 1979, Nr. 3, Seiten 14 - 23 und bei Bullesbach, Kontakte (Merck) 1980, Nr. 1, Seiten 23 - 35 beschrieben.

Gemäß der älteren europäischen Anmeldung mit der Offenlegungsnummer 0 287 882 werden Spacer der allgemeinen Formel I, in der R2 Wasserstoff oder einen Aminosäurerest, der mit einer schwach sauer oder bas sisch abspaltbaren Aminoschutzgruppe geschützt ist, bedeutet, beschrieben,

Die Erfindung betrifft auch ein Verfahren zur Herstellung der Verbindungen der Formel I, das dadurch gekennzeichnet ist, daß man
a) eine Verbindung der Formel II 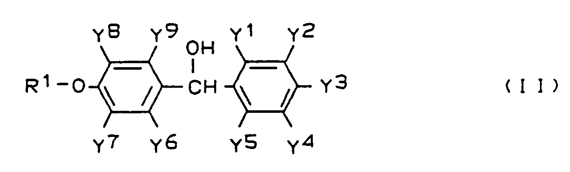 worin
   - R¹: (C₁-C₈)-Alkyl,
   - Y¹, Y², Y³, Y⁴, Y⁵, Y⁶, Y⁷, Y⁸, Y⁹: Wasserstoff, (C₁-C₄)-Alkyl, (C₁-C₄)-Alkoxy oder -O-(CH₂)ₙ-COOH, wobei die Reste gleich oder verschieden sein können, ein Rest aber -O-(CH₂)ₙ-COOH ist, oder
   - Y¹, Y², Y⁵, Y⁶, Y⁷, Y⁸, Y⁹: Wasserstoff, (C₁-C₄)-Alkyl oder (C₁-C₄)-Alkoxy, wobei die Reste gleich oder verschieden sein können, Y³ (C₁-C₈)-Alkoxy oder Wasserstoff und Y⁴ -(CH₂)ₙ-COOH oder -NH-CO-(CH₂)ₙ-COOH, und
   - n: eine ganze Zahl von 1 bis 6
   bedeuten, mit einer Verbindung der Formel III 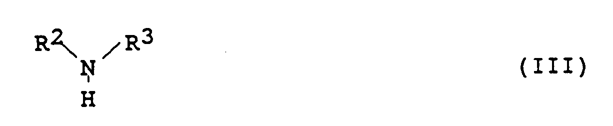 in welcher
   - R²: eine schwach sauer oder basisch abspaltbare Aminoschutzgruppe und
   - R³: Wasserstoff oder (C₁-C₄)-Alkyl
   bedeuten, umsetzt oder
b) eine Verbindung der Formel IV 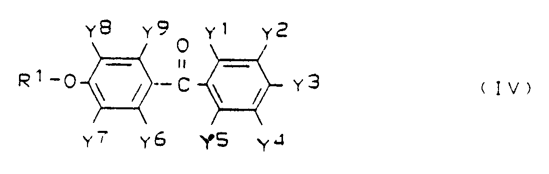 mit Hydroxylamin zu einer Verbindung der Formel V 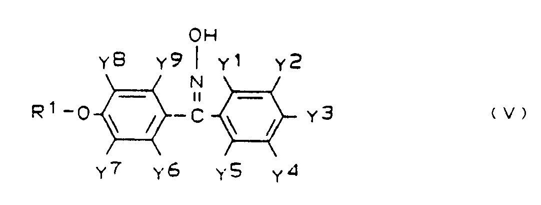 worin R¹, Y¹, Y², Y³, Y⁴, Y⁵, Y⁶, Y⁷, Y⁸ und Y⁹ wie oben definiert sind, umsetzt,
anschließend das Oxim zum Amin reduziert, vorzugsweise mit Zink in Eisessig oder mit Zn in ammoniakalischem Ethanol (S. Gaehde, G. Matsueda, Int. J. Peptide Protein Res. 18, 451 (1981)) und das Amin gegebenenfalls durch geeignete Reagenzien wie z.B. Chlorameisensäurederivate oder Kohlensäurederivate in Verbindungen der Formel I überführt, in der R² eine schwach saure oder basisch abspaltbare Aminoschutzgruppe und R³ Wasserstoff bedeuten.

Die Umsetzung einer Verbindung der Formel II mit einer Verbindung der Formel III führt man vorzugsweise in einem polaren, protischen Lösungsmittel, wie z.B. Essigsäure bei einer Temperatur zwischen 0 °C und dem Siedepunkt des Reaktionsgemisches durch.

Verbindungen der Formel II erhält man beispielsweise durch Reduktion von Benzophenon-Derivaten der Formel IV, 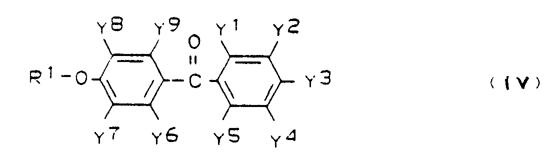
worin R¹, Y¹, Y², Y³, Y⁴, Y⁵, Y⁶, Y⁷, Y⁸ und Y⁹ wie oben definiert sind, mit geeigneten, d.h. für die Ketogruppe selektiven Reduktionsmitteln wie z.B. Natriumborhydrid.

Benzophenon-Derivate der Formel IV werden
a) durch Umsetzung von Benzophenonen der Formel IV worin R¹ wie oben definiert ist und Y¹, Y², Y³, Y⁴, Y⁵, Y⁶, Y⁷, Y⁸ und Y⁹ Wasserstoff, (C₁-C₄)-Alkyl, (C₁-C₄)-Alkoxy und einer der Reste Y¹-Y⁹ Hydroxyl bedeuten, mit ω-Halogenfettsäuren der Formel VI

   Hal-(CH₂)ₙ-COOH (VI)

   worin Hal Halogen bedeutet und n wie oben definiert ist, oder deren Ester, wobei im Falle der Ester anschließend eine alkalische Verseifung der Estergruppierung z.B. mit Natronlauge erfolgt (M. Prashad et al., Indian J. Chem. 17B, 496-498 (1979)),
b) z.B. durch Umsetzung von Benzoesäurechloride der Formel VII 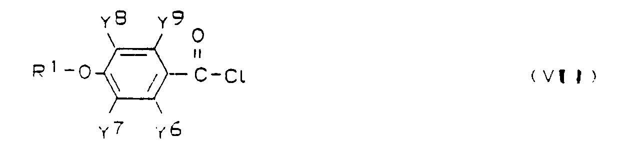 mit ω-Phenoxyalkansäuren der Formel VIII 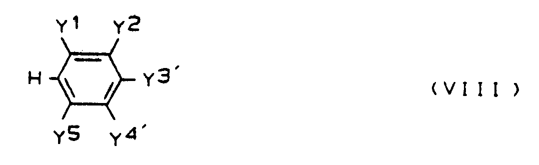 worin R¹, Y¹, Y², Y⁵, Y⁶, Y⁷, Y⁸ und Y⁹ wie unter a) definiert sind, Y^{3'} für -O-(CH₂)ₙ-COOR⁴ steht und Y^{4'} wie Y⁴ unter a) definiert ist oder Y^{3'} wie Y³ unter a) definiert ist und Y^{4'} für -(CH₂)ₙ-COOR⁴ steht, n wie unter a) definiert ist und R⁴ (C₁-C₈)-Alkyl, bevorzugt Methyl oder Ethyl bedeutet,
   unter Verwendung eines Lewis-Katalysators wie z.B. Aluminiumtrichlorid oder Titantetrachlorid (Organikum, 13. Auflage, S. 354 (1974)), oder
c) durch Umsetzung der entsprechenden Benzoesäurechloride der Formel VII mit entsprechend substituierten Phenolen der Formel IX zu den entsprechenden Phenylestern der Formel X, 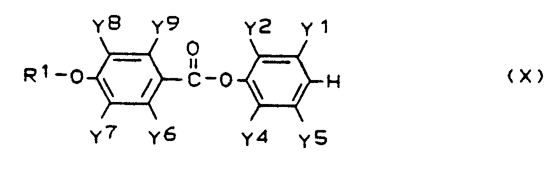 anschließende Fries-Verschiebung mit Lewis-Säuren wie z.B. Titantetrachlorid (R. Martin et al., Monatsh. Chemie 110, 1057-1066 (1979)) und weitere Umsetzung mit ω-Halogenfettsäuren der Formel VI, worin Hal und n wie oben definiert sind, erhalten.

Verbindungen der Formel VIII, worin Y^{3'} (C₁-C₈)-Alkoxy oder Wasserstoff und Y^{4'} -(CH₂)ₙ-COOR⁴ bedeuten, erhält man z.B., durch Umsetzung der entsprechenden Benzaldehyde in denen Y^{4'} für CHO steht mit Malonsäurehalbestern in Pyridin/Piperidin zu den entsprechenden Zimtsäureestern und anschließende Hydrierung. Man erhält in diesem Falle Verbindungen der Formel VIII mit n = 2.
Ebenfalls möglich ist die Umsetzung der entsprechenden Benzaldehyde mit α-Halogenessigsäureestern in Gegenwart von Zink, Dehydratisierung zu den Zimtsäureestern und anschließende Hydrierung.

Verbindungen der Formel VIII, worin Y^{3'} für -O-(CH₂)ₙ-COOR⁴ und Y^{4'} für Y⁴ stehen, stellt man durch Umsetzung der entsprechenden Phenole der Formel IX 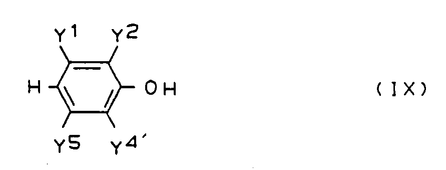
mit den entsprechenden ω-Halogenalkansäureestern, z.B. mit Natriumhydrid in Dimethylformamid (DMF) oder Kaliumcarbonat in Aceton her.

Analog können auch die der allgemeinen Formel I entsprechenden Verbindungen der Formeln VII, VIII, IX, und X hergestellt werden.

Die Erfindung betrifft weiterhin die Verwendung einer Verbindung der Formel I, bei der Festphasensynthese von Verbindungen der Formel XI

P―NH-R³ (XI)

in welcher P für einen Peptidrest aus α-Aminosäuren steht und R³ wie oben definiert ist sowie ein Verfahren zur Herstellung eines Peptids der Formel XI, in welcher P, R² und R³ wie oben definiert sind, durch Festphasensynthese, das dadurch gekennzeichnet ist, daß man eine Verbindung der Formel I mit in der Peptidchemie üblichen Kupplungsreagenzien über die -O-(CH₂)ₙ-COOH, -(CH₂)ₙ-COOH oder -NH-CO-(CH₂)ₙ-COOH Gruppierung an ein Harz kuppelt, die Schutzgruppe R² abspaltet, stufenweise durch basenlabile oder gegen schwache Säuren labile Aminoschutzgruppen temporär geschützte α-Aminosäuren gegebenenfalls in Form ihrer aktivierten Derivate ankuppelt und nach beendetem Aufbau das Peptid der Formel XI, durch Behandeln mit einer mittelstarken Säure von Harz freisetzt, wobei gleichzeitig oder durch geeignete Maßnahmen daran anschließend temporär eingeführte Seitenkettenschutzgruppen wieder abgespalten werden.

Falls zur Verhinderung von Nebenreaktionen oder für die Synthese spezieller Peptide erforderlich, sind die funktionellen Gruppen in der Seitenkette von Aminosäuren durch geeignete Schutzgruppen (siehe z.B. T.W. Greene, "Protective Groups in Organic Synthesis", New York, John Wiley & Sons, 1981) zusätzlich geschützt, wobei in erster Linie Arg(Tos), Arg(Mts), Arg(Mtr), Asp(OBzl), Asp(OBut), Cys(4-MeBzl), Cys(Acm), Cys(SBut), Glu(OBzl), Glu(OBut), His(Tos), His(Fmoc), His(Dnp), His(Trt), Lys(Cl-2), Lys(Boc), Met(O), Ser(Bzl), Ser(But), Thr(Bzl), Thr(But) eingesetzt werden.

Die als Trägermaterial verwendeten Harze sind kommerziell erhältlich oder selbst hergestellt, wie z.B. Alkoxybenzylalkoholharze, Aminomethylharze oder Benzhydrylaminoharze. Bevorzugt sind Aminomethyl-, Benzhydrylamino (BHA) und Methylbenzhydrylamino-Harze (MBHA). Die Beladung bestimmt man durch Aminosäureanalyse und/oder Elementaranalyse.

Als Kupplungsreagenz für die Verbindung der Formel I und die weiteren Aminosäurederivate können alle möglichen in der Peptidsynthese verwendeten Aktivierungs-Reagenzien, siehe z.B. Houben-Weyl, Methoden der organischen Chemie Band 15/2, verwendet werden, insbesondere aber Carbodiimide wie z.B. N,N′-Dicyclohexylcarbodiimid, N,N′-Diisopropylcarbodiimid oder N-Ethyl-N′-(3-dimethylaminopropyl)carbodiimid. Die Kupplung kann dabei direkt durch Addition von Aminosäurederivat mit dem Aktivierungsreagenz und gegebenenfalls einem die Racemisierung unterdrückenden Zusatz wie z.B. 4-Dimethylaminopyridin, 1-Hydroxybenzotriazol (HOBt) (W. König, R. Geiger, Chem. Ber. 103, 708 (1970)) oder 3-Hydroxy-4-oxo-3,4-dihydrobenzotriazin (HOObt) (W. König, R. Geiger, Chem. Ber. 103, 2054 (1970) zum Harz durchgeführt werden oder aber die Voraktivierung des Aminosäurederivats als symmetrisches Anhydrid oder HOBt- bzw. HOObt-Ester kann separat erfolgen und die Lösung der aktivierten Spezies in einem geeigneten Lösungsmittel zum kupplungsfähigen Peptidharz gegeben werden.

Die Kupplung bzw. Aktivierung der Verbindung der Formel I und der Aminosäurederivate mit einem der obengenannten Aktivierungsreagenzien kann in Dimethylformamid oder Methylenchlorid oder einer Mischung aus beiden durchgeführt werden. Das aktivierte Aminosäurederivat wird üblicherweise in einem 1,5- bis 4-fachen Überschuß eingesetzt. In Fällen, in denen eine unvollständige Kupplung eintritt, wird die Kupplungsreaktion wiederholt, ohne vorher die für die Kupplung der nächstfolgenden Aminosäure nötige Entblockung der α-Aminogruppe des Peptidharzes durchzuführen.

Der erfolgreiche Ablauf der Kupplungsreaktion kann mittels der Ninhydrin-Reaktion, wie z.B. von E. Kaiser et al. Anal. Biochem. 34 595 (1970) beschrieben, überprüft werden. Die Synthese kann auch automatisiert z.B. mit einem Peptid-Synthesizer Modell 430A der Fa. Applied Biosystems durchgeführt werden, wobei entweder die vom Gerätehersteller vorgesehenen Syntheseprogramme oder aber auch vom Benutzer selbst erstellte verwendet werden können. Letztere werden insbesondere bei der Verwendung von mit der Fmoc-Gruppe geschützten Aminosäurederivaten eingesetzt.

Die Abspaltung der Peptidamide vom Harz erfolgt durch Behandlung mit in der Peptidsynthese üblicherweise verwendeten mittelstarken Säuren (z.B. Trifluoressigsäure) wobei als Kationenfänger Substanzen wie Phenol, Kresol, Thiokresol, Anisol, Thioanisol, Ethandithiol, Dimethylsulfid, Ethylmethylsulfid oder ähnliche in der Festphasensynthese übliche Kationenfänger einzeln oder eine Mischung von zwei oder mehr dieser Hilfsmittel zugesetzt werden. Die Trifluoressigsäure kann dabei auch durch geeignete Lösungsmittel, wie z.B. Methylenchlorid, verdünnt angewendet werden.
Bei der Abspaltung des Spacers vom Harz erfolgt gleichzeitig die Abspaltung der Seitenkettenschutzgruppen.

Die Reinigung der so erhaltenen Rohpeptide erfolgt mittels Chromatographie an ®Sephadex, Ionenaustauscherharzen oder HPLC.

Die nachstehenden Beispiele dienen zur Erläuterung der vorstehenden Erfindung, ohne daß sie darauf beschränkt wäre.

### Beispiel 1

### 4-Phenoxybuttersäuremethylester

In 160 ml eisgekühltes, trockenes Methanol werden 16 ml Thionylchlorid eingetropft und anschließend 36 g Phenoxybuttersäure in Portionen zugegeben. Danach wird eine Stunde bei 40 °C gerührt. Dann wird das überschüssige Methanol abgezogen und der verbliebene Rückstand aus Petrolether (Kühlen auf -20 °C) umkristallisiert.
Ausbeute: 345 g, Schmelzpunkt: 255-26,5 °C

### Beispiel 2

### 4-(4′-Methoxybenzoyl)-phenoxybuttersäuremethylester

25,6 g wasserfreies Aluminiumtrichlorid werden in 64 ml 1,2-Dichlorethan gelöst und 28,6 g 4-Methoxybenzoylchlorid werden zugegeben. Dann werden unter heftigem Rühren 31 g 4-Phenoxybuttersäuremethylester langsam zugegeben. Man rührt noch 4 Stunden bei 50 °C, läßt abkühlen und gießt die Reaktionsmischung auf Eiswasser. Man trennt die ölige organische Schicht und die Wasserphase, wäscht die organische Phase mit Wasser und kristallisiert durch Zugabe von wenig Methanol. Das ausgefallene Produkt wird abgesaugt und aus Essigester umkristallisiert.
Ausbeute: 41 g, Schmelzpunkt: 117-118 °C

### Beispiel 3

### 4-(4′-Methoxybenzoyl)-phenoxybuttersäure

41 g 4-(4′-Methoxybenzoyl)-phenoxybuttersäuremethylester aus Beispiel 2 werden in 600 ml Dimethoxyethan/Wasser 4:1 gelöst und 63 ml 2N Natronlauge zugegeben. Man rührt 4 Stunden bei Raumtemperatur, säuert dann mit 3N Salzsäure auf pH 3 an und entfernt das organische Lösungsmittel im Vakuum.
Die ausgefallene Säure wird abfiltriert und im Vakuum über Phosphorpentoxid getrocknet.
Ausbeute: 38,7 g, Schmelzpunkt: 141-142 °C

### Beispiel 4

### (4-Carboxylatopropyloxyphenyl)-4′-methoxyphenylcarbinol

18,9 g 4-(4′-Methoxybenzoyl)-phenoxybuttersäure (Beispiel 3) werden in 600 ml Methanol bei 40 °C gelöst, 60 ml 1N Natronlauge und 3,4 g Natriumborhydrid portionsweise zugegeben. Man rührt noch eine Stunde bei 40 °C reduziert dann auf kleines Volumen und stellt mit 3N Salzsäure auf pH 2,8. Danach wird das Methanol am Rotationsverdampfer abgezogen, die wäßrige Mischung mit Ethylacetat extrahiert und die organische Lösung mit gesättigter Natriumchloridlösung gewaschen. Nach Trocknung über Natriumsulfat wird das Lösungsmittel entfernt. Es verbleiben 18,2 g einer amorphen Substanz, die sofort weiter umgesetzt wird.

### Allgemeine Vorschrift zur Kupplung von Fmoc-amid (9-Fluorenylmethylcarbamat) an Carbinolderivate

20 mmol Fmoc-amid und 20 mmol frisch hergestelltes Carbinol werden in 50 ml warmem Eisessig gelöst. Dann werden 5 Tropfen konzentrierte Schwefelsäure zugegeben und bei Raumtemperatur oder bei 40 °C gerührt. Das Fortschreiten der Reaktion wird mittels Dünnschichtchromatographie kontrolliert. Nach beendeter Reaktion wird Eisessiglösung auf Eiswasser gegossen, das ausgefallene Produkt abgesaugt und umkristallisiert.

### Beispiel 5

### N-Fmoc-(4-Carboxylatopropyloxyphenyl-4′-methoxyphenyl)methylamid

Nach obiger Vorschrift wird das unter Beispiel 4 hergestellte (4-Carboxylatopropyloxyphenyl)-4′-methoxyphenylcarbinol umgesetzt.
Ausbeute: 91 %, Schmelzpunkt: 186-187 °C

| Analyse: C₃₃H₃₁NO₆ | | | |
|---|---|---|---|
| berechnet: | C 73,73 | H 5,81 | N 2,61 |
| gefunden: | C 73,6 | H 6,0 | N 2,6 |

### Beispiel 6

### N-Fmoc-(4-Carboxylatomethyloxyphenyl-4′-methoxyphenyl)methylamid

Nach obiger Vorschrift wird das in der deutschen Patentanmeldung P 37 11 866.8 Beispiel 3 beschriebene (4-Carboxylatomethyloxyphenyl)-4′-methoxyphenylcarbinol umgesetzt.
Ausbeute: 60 %, Schmelzpunkt: 176-179 °C

| Analyse: C₃₁H₂₇NO₆ | | | |
|---|---|---|---|
| berechnet: | C 73,15 | H 5,35 | N 2,75 |
| gefunden: | C 73,3 | H 5,3 | N 2,95 |

### Beispiel 7

Synthese von
H-Leu-Gly-Gly-Gly-Gln-Gly-Lys-Val-Leu-Gly-NH₂
unter Verwendung des in Beispiel 5 beschriebenen Ankers. Die Synthese erfolgte in einem Peptidsynthesizer der Fa. Labotec.

Von 1,5 g Boc-Val-Harz (Beladung 0,76 mmol/g) wurde zunächst mit Trifluoressigsäure in Methylenchlorid die Schutzgruppe entfernt. Nach dem Waschen mit Dichlormethan und Ethyldiisopropylamin und wiederum Dichlormethan wurde das Harz getrocknet. Anschließend gab man 2,1 mmol des in Beispiel 5 hergestellten Ankers zusammen mit 3,15 mmol HOBt gelöst in 20 ml trockenem DMF auf das Harz und fügte dann 2,3 mmol Diisopropylcarbodiimid dazu. Unter langsamen Durchmischen ließ man bei Raumtemperatur über Nacht reagieren. Die Vollständigkeit der Reaktion wurde mit der Ninhydrin-Reaktion (Kaiser-Test) überprüft. Dann wurde das Harz abgesaugt, mit DMF gewaschen und anschließend das Peptid am Harz aufgebaut (für die Synthese wurden 1 g des oben hergestellten Harzes eingesetzt), wobei folgende Schritte cyclisch durchlaufen wurden.
- Abspaltung der Fmoc-Schutzgruppe mit 20 % Piperidin in DMF
- Waschen des Harzes mit DMF
- Aufkuppeln der Fmoc-Aminosäure unter in situ Aktivierung als HOBt-Ester unter Verwendung von Diisopropylcarbodiimid als Aktivierungsreagenz (1,5 mmol Aminosäure, 2,25 mmol HOBt, 1,6 mmol Diisopropylcarbodiimid)
Falls die Kupplung unvollständig war (Kaiser-Test) wurde der Kupplungsschritt wiederholt. Als letzte Aminosäure wurde Boc-Leu-OH eingesetzt.

Nach beendeter Synthese wurde das Harz mit DMF und Isopropanol gewaschen und im Hochvakuum getrocknet. Die Abspaltung erfolgte während einer Zeit von 2,5 Stunden bei Raumtemperatur mit einer Mischung aus 20 ml Trifluoressigsäure/15 ml Dichlormethan/5 ml Thioanisol/0,5 ml Ethandithiol. Anschließend wurde in Diethylether abgesaugt und mit 50 %iger Trifluoressigsäure in Dichlormethan nachgewaschen. Das ausgefallene Rohpeptid wurde abgesaugt und dreimal mit Diethylether gewaschen und im Hochvakuum getrocknet.
Ausbeute: 91 % Rohpeptid
Aminosäureanalyse: Gly 5,1; Glu 0,98; Leu 1,78; Val 0,81; Lys 1,01.

### Beispiel 8

### 2,4-Dimethoxyzimtsäureethylester

83 g 2,4-Dimethoxybenzaldehyd und 66 g Malonsäuremonoethylester werden in 100 ml trockenem Pyridin gelöst und nach Zugabe von 1 ml Piperidin und 40 g Molekularsieb unter Rückfluß erhitzt bis die CO₂-Entwicklung aufgehört hat. Das Reaktionsgemisch wird nach Abkühlen auf zerstoßenes Eis gegossen und mit konz. Salzsäure unter Rühren auf pH 1-2 gestellt. Man rührt noch 20 Minuten nach, saugt das ausgefallene Produkt ab, wäscht mit 1 N Salzsäure und Wasser und trocknet im Exsiccator.
Ausbeute: 100,2 g, Schmp. 58 °C

### Beispiel 9

### 2,4-Dimethoxyhydrozimtsäureethylester

100,2 g 2,4-Dimethoxyzimtsäureethylester werden in 400 ml Methanol gelöst und mit Palladium/Kohle bei Normaldruck hydriert. Man saugt vom Katalysator ab und engt im Vakuum ein.
Ausbeute: 94,7 g gelbliches Öl

### Beispiel 10

### 3-[2,4-Dimethoxy-5-(4-methoxybenzoyl)phenyl]propionsäureethylester

52 g Aluminiumtrichlorid werden in 200 ml 1,2-Dichlorethan suspendiert und auf 0 °C gekühlt. Anschließend werden bei dieser Temperatur 66,5 g 4-Methoxybenzoylchlorid zugetropft gefolgt von 94,7 g 2,4-Dimethoxyhydrozimtsäureethylester in 30 ml 1,2-Dichlorethan. Anschließend wird unter Feuchtigkeitsausschluß auf 50 °C erhitzt bis die Reaktion beendet ist. Danach wird die Reaktionsmischung auf Eiswasser gegossen, die organische Phase abgetrennt und nach Verdünnen mit 200 ml Methylenchlorid mit Natriumbicarbonat-Lösung und Wasser extrahiert. Man trocknet über Magnesiumsulfat und engt ein. Es verbleibt eine grau-braune Festsubstanz, die direkt weiter umgesetzt wird.
Ausbeute: 128,9 g, Schmp. 89 °C

### Beispiel 11

### 3-[2,4-Dimethoxy-5-(4-methoxybenzoyl)phenyl]propionsäure

55,8 g 3-[2,4-Dimethoxy-5-(4 methoxybenzoyl)phenyl]propionsäureethylester werden mit 300 ml 2N Natronlauge und 300 ml Dioxan versetzt und über Nacht gerührt. Dann wird das Dioxan abgezogen und die alkalische wäßrige Lösung einmal mit 100 ml Essigester extrahiert. Die Wasserphase wird mit konz. Salzsäure auf pH 2 gestellt wobei ein Teil des Produktes ausfällt. Man extrahiert dreimal mit je 200 ml Essigester, wäscht die organische Phase mit Wasser, trocknet über Natriumsulfat und engt ein. Der Rückstand wird aus Essigester/Aceton/Hexan umkristallisiert.
Ausbeute: 37 g, Schmp. 177-180 °C

### Beispiel 12

### 2,4-Dimethoxyzimtsäuremethylester

Eine Mischung aus 105,6 g 2,4-Dimethoxybenzaldehyd, 93,6 g Malonsäuremonomethylester-Kaliumsalz, 200 ml Pyridin (über KOH getrocknet), 1,2 ml Piperidin, 36 ml Essigsäure und 48 g Molekularsieb 3Å wird solange auf 150 °C erhitzt bis die CO₂-Entwicklung aufgehört hat.
Die Lösung wird warm auf Eis gegossen, mit konz. Salzsäure auf pH 1-2 angesäuert und noch 20 Minuten im Eisbad nachgerührt. Der Niederschlag wird abgesaugt, mit 1N HCl und Wasser nachgewaschen und getrocknet.
Ausbeute: 113,8 g, Schmelzpunkt: 78 °C

### Beispiel 13

### 2,4-Dimethoxyzimtsäure

In einem 11-Dreihalskolben mit Magnetrührer, Rückflußkühler und Innenthermometer werden 180 ml trockenes Pyridin vorgelegt und darin 124,9 g Malonsäure unter Rühren gelöst. Dann werden 166,18 g 2 4-Dimethoxybenzaldehyd in Portionen zugegeben, wobei die Innentemperatur auf 25 °C absinkt. Zu der erhaltenen Mischung gibt man 9,9 ml trockenes Piperidin und erwärmt dann langsam.
Die Badtemperatur wird auf 130 °C eingestellt. Nach 1,5 Stunden ist die CO₂-Entwicklung beendet und die Mischung siedet bei 107 °C.
Anschließend wird die Mischung heiß auf 1 kg Eis gegossen. Man säuert mit konz. HCl unter Rühren auf pH 1 an, rührt noch 20 Minuten unter Eiskühlung nach und saugt den Niederschlag ab. Man wäscht noch je 3mal mit je 200 ml 2N HCl und Wasser nach und trocknet. Der noch verbliebene Aldehyd wird durch suspendieren des Niederschlages in 600 ml heißem Cyclohexan und erneutes Absaugen entfernt. Man wäscht anschließend mit 100 ml heißem Cyclohexan nach und trocknet im Exsiccator. Aus dem Filtrat kristallisiert der Aldehyd bei Abkühlen aus.
Eine weitere Möglichkeit der Reinigung besteht in der Umkristallisation aus Methanol/Wasser. Dazu wird das Produkt heiß in möglichst wenig Methanol gelöst und mit Wasser bis zur Trübung versetzt. Nach Abkühlen fällt die Zimtsäure aus während der Aldehyd in Lösung bleibt.
Ausbeute: Zimtsäure 181,4 g (mit Cyclohexan behandelt),
Aldehyd 14,5 g (aus Cyclohexan ausgefallen),
Schmelzpunkt: 182-184 °C

### Beispiel 14

### 3-(2,4-Dimethoxyphenyl)propionsäuremethylester

20,8 g 2,4-Dimethoxyzimtsäure werden in einer Mischung aus 500 ml Methanol und 100 ml Essigester heiß gelöst und nach Zugabe von 1 g Katalysator (10 % Pd/C) bei Normaldruck hydriert. Die Wasserstoffaufnahme sollte nach 3 Stunden beendet sein. Falls die Hydrierung nicht vollständig ist wird nochmals frischer Katalysator zugesetzt.
Nach beendigter Reaktion wird der Katalysator abgetrennt, die Lösung auf ca. 200 ml eingeengt und 20 ml einer ca. 2,4 M methanolischen Salzsäure zugegeben und die Mischung 2 Tage bei Raumtemperatur stehengelassen. Die Veresterung ist dann vollständig. Man zieht im Vakuum das Methanol ab, nimmt den Rückstand in 200 ml Essigester auf, wäscht die organische Phase mit NaHCO₃-Lösung und gesättigter NaCl-Lösung (je 3 mal 50 ml), trocknet über Na₂SO₄ und engt ein.
Ausbeute: 20,0 g eines Öls

### Beispiel 15

### 3-(2,4-Dimethoxyphenyl)propionsäuremethylester

113,8 g 2,4-Dimethoxyzimtsäuremethylester werden unter Erwärmen in 1,5 l Methanol gelöst und an 5 g Katalysator (10 % Pd/C) hydriert. Nach Filtration des Katalysators wird eingeengt. Es verbleiben 113 g eines Öles, das direkt in die nachfolgende Friedel-Crafts-Acylierung eingesetzt wird.
Ausbeute: 113 g Öl

### Beispiel 16

### 3-[2,4-Dimethoxy-5-(4-methoxybenzoyl)phenyl]propionsäuremethylester

36,6 g Aluminiumtrichlorid werden in 130 ml 1,2-Dichlorethan gelöst und 42,65 g 4-Methoxybenzoylchlorid zur auf 0 °C gekühlten Lösung zugegeben. Bei dieser Temperatur wird anschließend 56 g 3-(2,4-Dimethoxyphenyl)propionsäuremethylester (Beispiel 17) gelöst in 30 ml 1,2-Dichlorethan zugetropft und dann unter Feuchtigkeitsausschluß bis zur Beendigung der Reaktion (ca. 36 Stunden) bei 50 °C gerührt. Die abgekühlte Reaktionsmischung wird mit 200 ml Wasser versetzt, mit 400 ml CH₂Cl₂ verdünnt mit Bicarbonat und Wasser extrahiert, über Natriumsulfat getrocknet und eingeengt. Man löst in Diisopropylether und fällt mit Hexan. Nach Absaugen erhält man 62,1 g und aus der Mutterlauge weitere 10,5 g des Produktes.
Ausbeute 72,6 g, Schmelzpunkt 114-117 °C

### Beispiel 17

### 3-[2,4-Dimethoxy-5-(4-methoxybenzoyl)phenyl]propionsäure

60,9 g 3-[2,4-Dimethoxy-5-(4-methoxybenzoyl)phenyl]propionsäuremethylester werden mit 300 ml 2N Natronlauge und 300 ml Dioxan versetzt und über Nacht gerührt. Dann wird das Dioxan abgezogen und die alkalische wäßrige Lösung einmal mit 100 ml Essigester extrahiert. Die Wasserphase wird mit konz. Salzsäure auf pH 2 gestellt wobei ein Teil des Produktes ausfällt. Man extrahiert dreimal mit je 200 ml Essigester, wäscht die organische Phase mit Wasser, trocknet über Natriumsulfat und engt ein. Der Rückstand wird aus Essigester/Aceton/Hexan umkristallisiert.
Ausbeute: 51,3 g, Schmelzpunkt: 177-180 °C

### Beispiel 18

### 5-Carboxylatoethyl-2,4-dimethoxy-4′-methoxybenzophenonoxim

37 g 3-[2,4-Dimethoxy-5-(4-methoxybenzoyl)phenyl]propionsäure, 22,4 g Hydroxylamin · Hydrochlorid und 21 g Natriumacetat (wasserfrei) werden in 300 ml absoluten Ethanol gegeben und unter Feuchtigkeitsausschluß 24 Stunden unter Rückfluß erhitzt. Man saugt von Ungelöstem ab, engt ein und versetzt den Rückstand mit Wasser. Dann wird mit 1N Salzsäure auf pH 2 gestellt und mit Essigester extrahiert. Die organische Phase wird mit Wasser gewaschen, über Magnesiumsulfat getrocknet und eingeengt. Der Rückstand wird aus Essigester/Hexan umkristallisiert.
Ausbeute: 34,3 g, Schmp. 124-129 °C

### Beispiel 19

### N-(9-Fluorenylmethoxycarbonyl)-[5-carboxylatoethyl-2,4-dimethoxyphenyl)-4′-methoxyphenyl]methylamin

34 g 5-Carboxylatoethyl-2,4-dimethoxy-4′-methoxybenzophenonoxim werden in eine Mischung von 100 ml Ethanol und 500 ml 25 %iger Ammoniaklösung gegeben, dann werden 29,5 g Zinkpulver zugegeben und die Mischung über Nacht bei 50 °C gerührt. Dann werden weitere 30 g Zink und 260 ml 25 %iger Ammoniak zugegeben und weitere 24 Stunden bei 50 °C gerührt. Man saugt heiß vom Zink ab und zieht Ethanol ab. Die gekühlte wäßrige Phase wird mit konz. Salzsäure neutral gestellt und einmal mit Essigester extrahiert.
Das erhaltene Amin wird nicht isoliert sondern die wäßrige Lösung direkt weiter umgesetzt. Dazu stellt man die Lösung mit festem Natriumhydrogencarbonat auf pH 8, verdünnt mit 250 ml Tetrahydrofuran und gibt 31 g 9-Fluorenylmethylsuccinimidylcarbonat hinzu und rührt über Nacht. Dann wird von Ungelöstem abfiltriert, das Tetrahydrofuran im Vakuum abgezogen und die wäßrige Phase mit 1N Schwefelsäure sauer gestellt. Man extrahiert mit Essigester, wäscht die organische Phase mit gesättigter Natriumchlorid-Lösung, trocknet über Magnesiumsulfat und engt ein. Der Rückstand wird in Essigester heiß gelöst, nach kurzem Abkühlen mit Ether und dann bis zur Trübung mit Hexan versetzt. Man läßt über Nacht im Kühlraum stehen, saugt das ausgefallene Produkt ab und kristallisiert nochmals in der beschriebenen Weise.
Ausbeute: 37,1 g, Schmp. 149-151 °C
Alternativ kann man das Produkt auch durch katalytische Hydrierung des Oxims an 10 % Palladium/Kohle in ammoniakalischem Ethanol und nachfolgende Umsetzung mit 9-Fluorenylmethylsuccinimidylcarbonat wie oben beschrieben erhalten.

### Beispiel 20

### Herstellung des Fmoc-Amid-Harzes

30,7 g aminomethyliertes Polystyrol-Harz (Beladung 1,04 mMol NH₂/g) und 28 g (49,5 mMol) N-(9-Fluorenylmethyloxycarbonyl)-[(5-carboxylatoethyl-2,4-dimethoxy-phenyl)-4′-methoxyphenyl]methylamin werden in einer Mischung aus 210 ml Dimethylformamid und 90 ml Dichlormethan suspendiert. Es werden 6,4 g HOObt und 23 ml Diisopropylcarbodiimid zugegeben und die Mischung über Nacht geschüttelt. Danach war die Reaktion beendet und das Harz wurde abgesaugt, mit DMF, DCM und MTB-Ether gewaschen. Nach Trocknung im Hochvakuum erhielt man 46,5 g Produkt mit einer Beladung von 0,57 mMol/g (bestimmt durch Elementaranalyse) bzw. 0,54 mMol/g (bestimmt durch Testabspaltung von Fmoc-NH₂ und Quantifizierung über HPLC).

### Peptid-Synthese

Für die Peptid-Synthese wurde folgendes Reaktionsschema angewendet:
Abspaltung der Fmoc-Schutzgruppe mit 20 % Piperidin in DMF, Waschen mit NMP, Aufkuppeln der nachfolgenden Fmoc-Aminosäure als in NMP gelöster HOObt-Ester oder als in DMF oder NMP mit Diisopropylcarbodiimid in situ voraktivierter HOObt-Ester, Waschen mit NMP. Dieser Zyklus wurde durchlaufen bis das Peptid am Harz aufgebaut war. Die Effektivität der Kupplung kann mittels Ninhydrin-Test verfolgt werden.

### Beispiel 21

### pGlu-His-Trp-Ser-Tyr-Gly-Leu-Arg-Pro-Gly-NH₂

Das Peptid wurde an 877 mg des oben hergestellten Harzes synthetisiert. Die Abspaltung des Rohpeptids erfolgte mittels TFA/Ethandithiol/Thioanisol 90/5/5 bei Raumtemperatur innerhalb 1 Stunde. Man erhielt 450 mg Rohprodukt, das mit einer authentischen Probe im HPLC identisch war.
FAB-MS (G, TFA): 1182 (M + H⁺)

### Beispiel 22

### H-Leu-Leu-Gln-Gly-Leu-Val-NH₂

Das Peptid wurde an 877 mg des oben hergestellten Harzes synthetisiert. Die Abspaltung des Rohpeptids erfolgte mittels TFA/Wasser 95/5 bei Raumtemperatur innerhalb 1 Stunde. Man erhielt aus 100 mg Harz 38 mg Rohprodukt, das mit einer authentischen Probe identisch war.
FAB-MS (3-NBA): 641 (M + H⁺)

### Beispiel 23

### H-Ile-Pro-Glu-Tyr-Leu-Gln-OH

Die Synthese dieses Peptides erfolgte wie oben beschrieben an 877 mg Harz. Als erste Aminosäure wurde Fmoc-Glu-OtBu aufgekuppelt. Dieses ergibt nach der Abspaltung vom Harz mit 95 % TFA/Wasser das Peptid mit C-terminalem Glutamin.

## Patentansprüche (Patentansprüche für folgende(n) Vertragsstaat(en): AT, BE, CH, DE, FR, GB, GR, IT, LI, LU, NL, SE)

1. Verbindung der allgemeinen Formel I 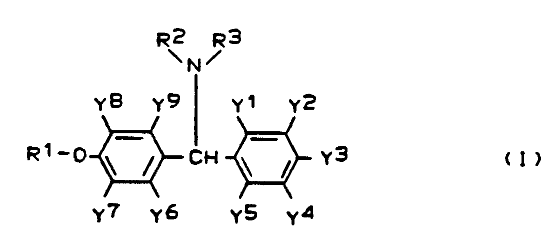 worin
R¹ (C₁-C₈)-Alkyl,
R² eine schwach sauer oder basisch abspaltbare Aminoschutzgruppe,
R³ Wasserstoff oder (C₁-C₄)-Alkyl,
Y¹, Y², Y³, Y⁴, Y⁵, Y⁶, Y⁷, Y⁸, Y⁹ Wasserstoff, (C₁-C₄)-Alkyl, (C₁-C₄)-Alkoxy oder -O-(CH₂)ₙ-COOH, wobei die Reste gleich oder verschieden sein können, ein Rest aber -O-(CH₂)ₙ-COOH ist, oder
Y¹, Y², Y⁵, Y⁶, Y⁷, Y⁸, Y⁹ Wasserstoff, (C₁-C₄)-Alkyl oder (C₁-C₄)-Alkoxy, wobei die Reste gleich oder verschieden sein können, Y³ (C₁-C₈)-Alkoxy oder Wasserstoff und Y⁴ -(CH₂)ₙ-COOH oder -NH-CO-(CH₂)ₙ-COOH, und
n eine ganze Zahl von 1 bis 6
bedeuten.

2. Verbindung der Formel I gemäß Anspruch 1, in welcher R¹ Methyl und n eine ganze Zahl von 1, 2 oder 3 sind.

3. Verbindung der Formel I gemäß einem oder mehreren der Ansprüche 1 bis 2, in welcher R² eine Urethanschutzgruppe und R³ Wasserstoff bedeuten.

4. Verbindung der Formel I gemäß einem oder mehreren der Ansprüche 1 bis 3 in welcher die Reste Y¹-Y⁹ für Methyl oder Methoxy stehen, wobei jedoch ein Rest -O-(CH₂)ₙ-COOH und mindestens 4 dieser Reste Wasserstoff sind oder die Reste Y¹, Y², Y⁵-Y⁹ für Methyl oder Methoxy stehen, wobei jedoch mindestens 4 dieser Reste Wasserstoff sind, Y³ für Methyl und Y⁴ für -(CH₂)ₙ-COOH stehen.

5. Verbindung der Formel I gemäß einem oder mehreren der Ansprüche 1 bis 4, in welcher ein Rest Y¹, Y³, Y⁵, Y⁷ oder Y⁸ für -O-(CH₂)ₙ-COOH steht.

6. Verbindung der Formel I gemäß einem oder mehreren der Ansprüche 1 bis 4, in welcher Y⁴ -(CH₂)ₙ-COOH bedeutet, Y³ (C₁-C₄)-Alkoxy und n = 2 sind, oder Y⁴ für -NH-CO-(CH₂)n-COOH steht, Y¹ und Y³ Methoxy und n = 2 bedeuten.

7. Verfahren zur Herstellung einer Verbindung der Formel I gemäß einem oder mehreren der Ansprüche 1 bis 6, dadurch gekennzeichnet, daß man
a) eine Verbindung der Formel II 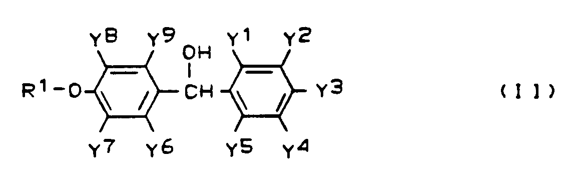 worin
R¹ (C₁-C₈)-Alkyl,
Y¹, Y², Y³, Y⁴, Y⁵, Y⁶, Y⁷, Y⁸, Y⁹ Wasserstoff, (C₁-C₄)-Alkyl, (C₁-C₄)-Alkoxy oder -O-(CH₂)ₙ-COOH, wobei die Reste gleich oder verschieden sein können, ein Rest aber -O-(CH₂)ₙ-COOH ist, oder
Y¹, Y², Y⁵, Y⁶, Y⁷, Y⁸, Y⁹ Wasserstoff, (C₁-C₄)-Alkyl oder (C₁-C₄)-Alkoxy, wobei die Reste gleich oder verschieden sein können, Y³ (C₁-C₈)-Alkoxy oder Wasserstoff und Y⁴ -(CH₂)ₙ-COOH oder -NH-CO-(CH₂)ₙ-COOH, und
n eine ganze Zahl von 1 bis 6
bedeuten, mit einer Verbindung der Formel III 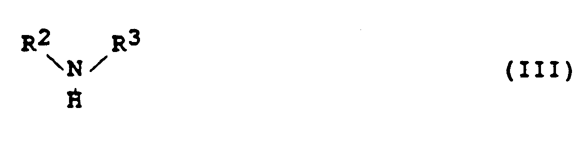 in welcher
R² eine schwach sauer oder basisch abspaltbare Aminoschutzgruppe und
R³ Wasserstoff oder (C₁-C₄)-Alkyl
bedeuten, umsetzt oder
b) eine Verbindung der Formel IV 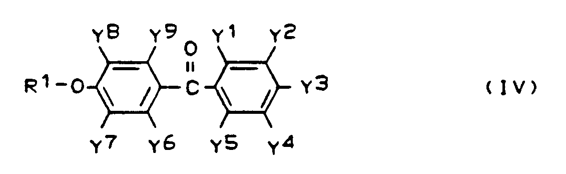 mit Hydroxylamin zu einer Verbindung der Formel V 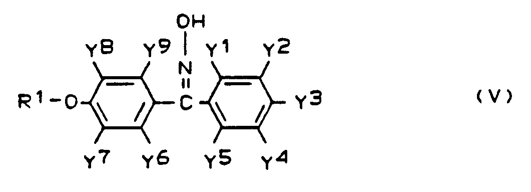 worin R¹, Y¹, Y², Y³, Y⁴, Y⁵, Y⁶, Y⁷, Y⁸ und Y⁹ wie oben definiert sind, umsetzt,
anschließend das Oxim zum Amin reduziert, das Amin gegebenenfalls in Verbindungen der Formel I überführt, in der R² eine schwach saure oder basisch abspaltbare Aminoschutzgruppe und R³ Wasserstoff bedeuten.

8. Verwendung einer Verbindung der Formel I gemäß einem oder mehreren der Ansprüche 1 bis 6, bei der Festphasensynthese von Verbindungen der Formel XI
P―NH-R³ (XI)
in welcher P für einen Peptidrest aus α-Aminosäuren steht und R³ wie in Anspruch 1 definiert ist.

9. Verfahren zur Herstellung eines Peptids der Formel XI, in welcher P und R³ wie in Anspruch 8 definiert sind, durch Festphasensynthese, dadurch gekennzeichnet, daß man eine Verbindung der Formel I mit in der Peptidchemie üblichen Kupplungsreagenzien über die -O-(CH₂)ₙ-COOH, -(CH₂)ₙ-COOH oder -NH-CO-(CH₂)ₙ-COOH Gruppierung an ein Harz kuppelt, die Schutzgruppe R² abspaltet, stufenweise durch basenlabile oder gegen schwache Säuren labile Aminoschutzgruppen temporär geschützte α-Aminosäuren gegebenenfalls in Form ihrer aktivierten Derivate ankuppelt und nach beendetem Aufbau das Peptid der Formel XI, durch Behandeln mit einer mittelstarken Säure von Harz freisetzt, wobei gleichzeitig oder durch geeignete Maßnahmen daran anschließend temporär eingeführte Seitenkettenschutzgruppen wieder abgespalten werden.

## Patentansprüche (Patentansprüche für folgende(n) Vertragsstaat(en): ES)

1. Verfahren zur Herstellung einer Verbindung der allgemeinen Formel I 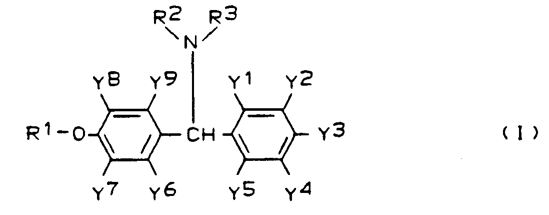 worin
R¹ (C₁-C₈)-Alkyl,
R² eine schwach sauer oder basisch abspaltbare Aminoschutzgruppe,
R³ Wasserstoff oder (C₁-C₄)-Alkyl,
Y¹, Y², Y³, Y⁴, Y⁵, Y⁶, Y⁷, Y⁸, Y⁹ Wasserstoff, (C₁-C₄)-Alkyl, (C₁-C₄)-Alkoxy oder -O-(CH₂)ₙ-COOH, wobei die Reste gleich oder verschieden sein können, ein Rest aber -O-(CH₂)ₙ-COOH ist, oder
Y¹, Y², Y⁵, Y⁶, Y⁷, Y⁸, Y⁹ Wasserstoff, (C₁-C₄)-Alkyl oder (C₁-C₄)-Alkoxy, wobei die Reste gleich oder verschieden sein können, Y³ (C₁-C₈)-Alkoxy oder Wasserstoff und Y⁴ -(CH₂)ₙ-COOH oder -NH-CO-(CH₂)ₙ-COOH, und
n eine ganze Zahl von 1 bis 6
bedeuten, dadurch gekennzeichnet, daß man
a) eine Verbindung der Formel II 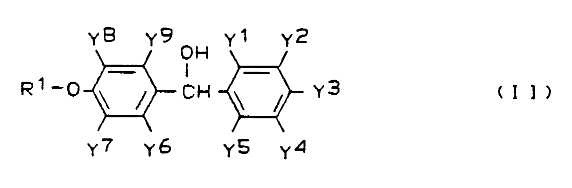 worin
R¹ (C₁-C₈)-Alkyl,
Y¹, Y², Y³, Y⁴, Y⁵, Y⁶, Y⁷, Y⁸, Y⁹ Wasserstoff, (C₁-C₄)-Alkyl, (C₁-C₄)-Alkoxy oder -O-(CH₂)ₙ-COOH, wobei die Reste gleich oder verschieden sein können, ein Rest aber -O-(CH₂)ₙ-COOH ist, oder
Y¹, Y², Y⁵, Y⁶, Y⁷, Y⁸, Y⁹ Wasserstoff, (C₁-C₄)-Alkyl oder (C₁-C₄)-Alkoxy, wobei die Reste gleich oder verschieden sein können, Y³ (C₁-C₈)-Alkoxy oder Wasserstoff und Y⁴ -(CH₂)ₙ-COOH oder -NH-CO-(CH₂)ₙ-COOH, und
n eine ganze Zahl von 1 bis 6
bedeuten,
mit einer Verbindung der Formel III in welcher 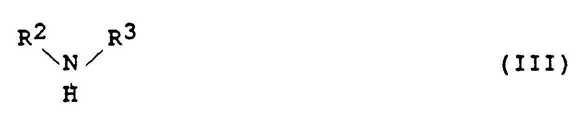
R² eine schwach sauer oder basisch abspaltbare Aminoschutzgruppe und
R³ Wasserstoff oder (C₁-C₄)-Alkyl
bedeuten, umsetzt oder
b) eine Verbindung der Formel IV 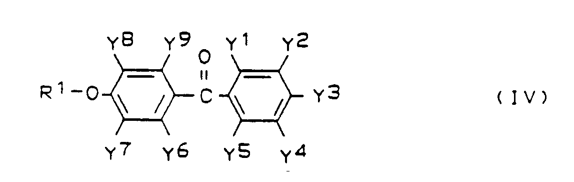 mit Hydroxylamin zu einer Verbindung der Formel V 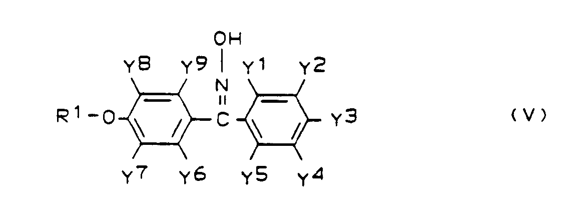 worin R¹, Y¹, Y², Y³, Y⁴, Y⁵, Y⁶, Y⁷, Y⁸ und Y⁹ wie oben definiert sind, umsetzt,
anschließend das Oxim zum Amin reduziert, das Amin gegebenenfalls in Verbindungen der Formel I überführt, in der R² eine schwach saure oder basisch abspaltbare Aminoschutzgruppe und R³ Wasserstoff bedeuten.

2. Verfahren gemäß Anspruch 1, dadurch gekennzeichnet, daß man eine Verbindung der Formel I herstellt, in welcher R¹ Methyl und n eine ganze Zahl von 1, 2 oder 3 sind.

3. Verfahren gemäß einem oder mehreren der Ansprüche 1 bis 2, dadurch gekennzeichnet, daß man eine Verbindung der Formel I herstellt, in welcher R² eine Urethanschutzgruppe und R³ Wasserstoff bedeuten.

4. Verfahren gemäß einem oder mehreren der Ansprüche 1 bis 3, dadurch gekennzeichnet, daß man eine Verbindung der Formel I herstellt, in welcher die Reste Y¹-Y⁹ für Methyl oder Methoxy stehen, wobei jedoch ein Rest -O-(CH₂)ₙ-COOH und mindestens 4 dieser Reste Wasserstoff sind oder die Reste Y¹, Y², Y⁵-Y⁹ für Methyl oder Methoxy stehen, wobei jedoch mindestens 4 dieser Reste Wasserstoff sind, Y³ für Methoxy und Y⁴ für -(CH₂)ₙ-COOH stehen.

5. Verfahren gemäß einem oder mehreren der Ansprüche 1 bis 4, dadurch gekennzeichnet, daß man eine Verbindung der Formel I herstellt, in welcher ein Rest Y¹, Y³, Y⁵, Y⁷ oder Y⁸ für -O-(CH₂)ₙ-COOH steht.

6. Verfahren gemäß einem oder mehreren der Ansprüche 1 bis 4, dadurch gekennzeichnet, daß man eine Verbindung der Formel I herstellt, in welcher Y⁴ -(CH₂)ₙ-COOH bedeutet, Y³ (C₁-C₄)-Alkoxy und n = 2 sind.

7. Verfahren zur Herstellung eines Peptids der Formel XI,
P―NH-R³ (XI)
in welcher P für einen Peptidrest aus α-Aminosäuren steht, R³ wie in Anspruch 1 definiert ist, durch Festphasensynthese, dadurch gekennzeichnet, daß man eine Verbindung der Formel I mit in der Peptidchemie üblichen Kupplungsreagenzien über die -O-(CH₂)ₙ-COOH oder -(CH₂)ₙ-COOH Gruppierung an ein Harz kuppelt, die Schutzgruppe R² abspaltet, stufenweise durch basenlabile oder gegen schwache Säuren labile Aminoschutzgruppen temporär geschützte α-Aminosäuren gegebenenfalls in Form ihrer aktivierten Derivate ankuppelt und nach beendetem Aufbau das Peptid der Formel XI, durch Behandeln mit einer mittelstarken Säure von Harz freisetzt, wobei gleichzeitig oder durch geeignete Maßnahmen daran anschließend temporär eingeführte Seitenkettenschutzgruppen wieder abgespalten werden.

## Claims (Claims for the following Contracting State(s): AT, BE, CH, DE, FR, GB, GR, IT, LI, LU, NL, SE)

1. A compound of the formula I 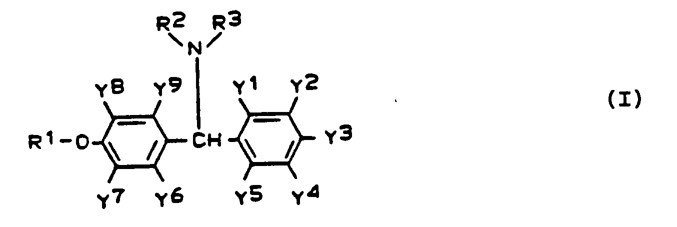 in which
R₁ denotes (C₁-C₈)-alkyl,
R² denotes an amino protective group which can be eliminated with weak acid or base,
R³ denotes hydrogen or (C₁-C₄)-alkyl,
Y¹, Y², Y³, Y⁴, Y⁵, Y⁶, Y⁷, Y⁸ and Y⁹ denote hydrogen, (C₁-C₄)-alkyl, (C₁-C₄)-alkoxy or -O-(CH₂)ₙ-COOH, where the radicals can be identical or different but one radical is -O-(CH₂)ₙ-COOH, or
Y¹, Y², Y⁵, Y⁶, Y⁷, Y⁸ and Y⁹ denote hydrogen, (C₁-C₄)-alkyl or (C₁-C₄)-alkoxy, where the radicals can be identical or different, Y³ denotes (C₁-C₈)-alkoxy or hydrogen and Y⁴ denotes -(CH₂)ₙ-COOH or -NH-CO-(CH₂)ₙ-COOH, and
n denotes an integer from 1 to 6.

2. A compound of the formula I as claimed in claim 1, in which R¹ is methyl and n is an integer from 1 to 3.

3. A compound of the formula I as claimed in one or both of claims 1 and 2, in which R² denotes a urethane protective group and R³ denotes hydrogen.

4. A compound of the formula I as claimed in one or more of claims 1 to 3, in which the radicals Y¹-Y⁹ represent methyl or methoxy, with, however, one radical being -O-(CH₂)ₙ-COOH and at least 4 of these radicals being hydrogen, or the radicals Y¹, Y² and Y⁵-Y⁹ represent methyl or methoxy, with, however, at least 4 of these radicals being hydrogen, Y³ represents methyl and Y⁴ represents -(CH₂)ₙ-COOH.

5. A compound of the formula I as claimed in one or more of claims 1 to 4, in which one radical Y¹, Y³, Y⁵, Y⁷ or Y⁸ represents -O-(CH₂)ₙ-COOH.

6. A compound of the formula I as claimed in one or more of claims 1 to 4, in which Y⁴ denotes -(CH₂)ₙ-COOH, Y³ is (C₁-C₄)-alkoxy and n is 2 or Y⁴ represents -NH-CO-(CH₂)ₙ-COOH, Y¹ and Y³ denote methoxy and n denotes 2.

7. A process for the preparation of a compound of the formula I as claimed in one or more of claims 1 to 6, which comprises
a) reacting a compound of the formula II 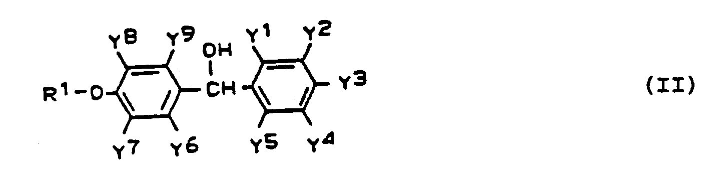 in which
R₁ denotes (C₁-C₈)-alkyl,
Y¹, Y², Y³, Y⁴, Y⁵, Y⁶, Y⁷, Y⁸ and Y⁹ denote hydrogen, (C₁-C₄)-alkyl, (C₁-C₄)-alkoxy or -O-(CH₂)ₙ-COOH, where the radicals can be identical or different but one radical is -O-(CH₂)ₙ-COOH, or
Y¹, Y², Y⁵, Y⁶, Y⁷, Y⁸ and Y⁹ denote hydrogen, (C₁-C₄)-alkyl or (C₁-C₄)-alkoxy, where the radicals can be identical or different, Y³ denotes (C₁-C₈)-alkoxy or hydrogen and Y⁴ denotes -(CH₂)ₙ-COOH or -NH-CO-(CH₂)ₙ-COOH, and
n denotes an integer from 1 to 6,
with a compound of the formula III 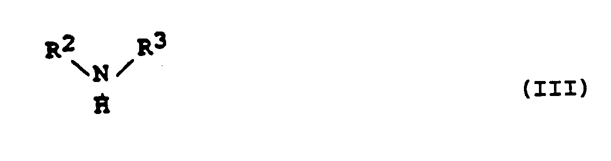 in which
R² denotes an amino protective group which can be eliminated with weak acid or base,
R³ denotes hydrogen or (C₁-C₄)-alkyl,
or
b) reacting a compound of the formula IV 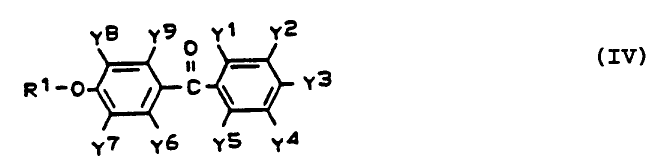 with hydroxylamine to give a compound of the formula V 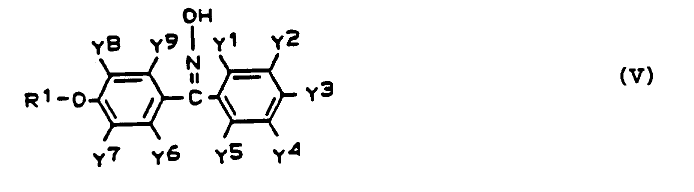 in which R¹, Y¹, Y², Y³, Y⁴, Y⁵, Y⁶, Y⁷, Y⁸ and Y⁹ are as defined above,
subsequently reducing the oxime to the amine, and, where appropriate, converting the amine into compounds of the formula I in which R² denotes an amino protective group which can be eliminated with weak acid or base, and R³ denotes hydrogen.

8. The use of a compound of the formula I as claimed in one or more of claims 1 to 6 in the solid-phase synthesis of compounds of the formula XI
P-NH-R³ (XI)
in which P represents a peptide residue composed of α-amino acids and R³ is as defined in claim 1.

9. A process for the preparation of a peptide of the formula XI, in which P and R³ are as defined in claim 8, by solid-phase synthesis, which comprises coupling a compound of the formula I, using coupling reagents customary in peptide chemistry, via the -O-(CH₂)₂-COOH, -(CH₂)ₙ-COOH or -NH-CO-(CH₂)ₙ-COOH group to a resin, eliminating the protective group R², coupling on stepwise α-amino acids which are temporarily protected by amino protective groups which are base-labile or labile to weak acids, which amino acids are, where appropriate, in the form of their activated derivatives, and, after synthesis is complete, liberating the peptide of the formula XI from the resin by treatment with a moderately strong acid, with temporarily introduced side-chain protective groups being eliminated again simultaneously or by suitable measures subsequent thereto.

## Claims (Claims for the following Contracting State(s): ES)

1. A process for the preparation of a compound of the formula I 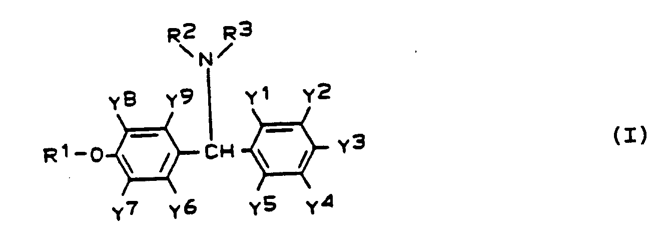 in which
R₁ denotes (C₁-C₈)-alkyl,
R² denotes an amino protective group which can be eliminated with weak acid or base,
R³ denotes hydrogen or (C₁-C₄)-alkyl,
Y¹, Y², Y³, Y⁴, Y⁵, Y⁶, Y⁷, Y⁸ and Y⁹ denote hydrogen, (C₁-C₄)-alkyl, (C₁-C₄)-alkoxy or -O-(CH₂)ₙ-COOH, where the radicals can be identical or different but one radical is -O-(CH₂)ₙ-COOH, or
Y¹, Y², Y⁵, Y⁶, Y⁷, Y⁸ and Y⁹ denote hydrogen, (C₁-C₄)-alkyl or (C₁-C₄)-alkoxy, where the radicals can be identical or different, Y³ denotes (C₁-C₈)-alkoxy or hydrogen and Y⁴ denotes -(CH₂)ₙ-COOH or -NH-CO-(CH₂)ₙ-COOH, and
n denotes an integer from 1 to 6,
which comprises
a) reacting a compound of the formula II 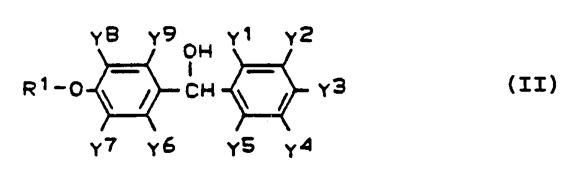 in which
R₁ denotes (C₁-C₈)-alkyl,
Y¹, Y², Y³, Y⁴, Y⁵, Y⁶, Y⁷, Y⁸ and Y⁹ denote hydrogen, (C₁-C₄)-alkyl, (C₁-C₄)-alkoxy or -O-(CH₂)ₙ-COOH, where the radicals can be identical or different but one radical is -O-(CH₂)ₙ-COOH, or
Y¹, Y², Y⁵, Y⁶, Y⁷, Y⁸ and Y⁹ denote hydrogen, (C₁-C₄)-alkyl or (C₁-C₄)-alkoxy, where the radicals can be identical or different, Y³ denotes (C₁-C₈)-alkoxy or hydrogen and Y⁴ denotes -(CH₂)ₙ-COOH or -NH-CO-(CH₂)ₙ-COOH, and
n denotes an integer from 1 to 6,
with a compound of the formula III 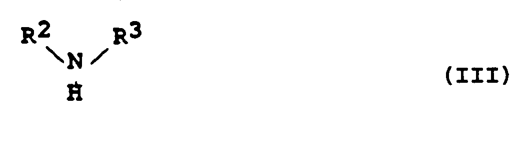 in which
R² denotes an amino protective group which can be eliminated with weak acid or base,
R³ denotes hydrogen or (C₁-C₄)-alkyl,
or
b) reacting a compound of the formula IV 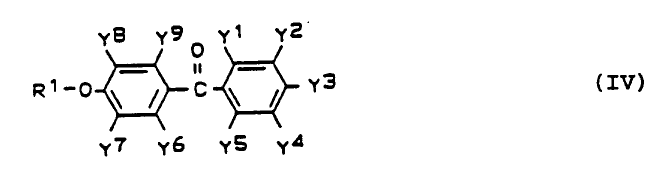 with hydroxylamine to give a compound of the formula V 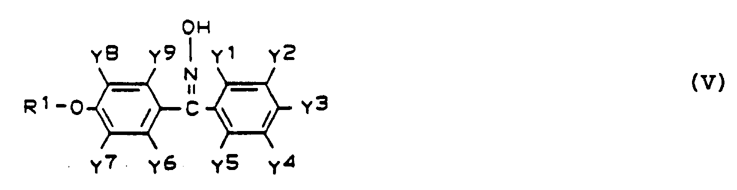 in which R¹, Y¹, Y², Y³, Y⁴, Y⁵, Y⁶, Y⁷, Y⁸ and Y⁹ are as defined above,
subsequently reducing the oxime to the amine, and, where appropriate, converting the amine into compounds of the formula I in which R² denotes an amino protective group which can be eliminated with weak acid or base, and R³ denotes hydrogen.

2. The process as claimed in claim 1, which comprises preparing a compound of the formula I in which R¹ is methyl and n is an integer from 1 to 3.

3. The process as claimed in one or both of claims 1 and 2, which comprises preparing a compound of the formula I in which R² denotes a urethane protective group and R³ denotes hydrogen.

4. The process as claimed in one or more of claims 1 to 3, which comprises preparing a compound of the formula I in which the radicals Y¹-Y⁹ represent methyl or methoxy, with, however, one radical being -O-(CH₂)ₙ-COOH and at least 4 of these radicals being hydrogen, or the radicals Y¹, Y² and Y⁵-Y⁹ represent methyl or methoxy, with, however, at least 4 of these radicals being hydrogen, Y³ represents methoxy and Y⁴ represents -(CH₂)ₙ-COOH.

5. The process as claimed in one or more of claims 1 to 4, which comprises preparing a compound of the formula I in which one radical Y¹, Y³, Y⁵, Y⁷, or Y⁸ represents -O-(CH₂)ₙ-COOH.

6. The process as claimed in one or more of claims 1 to 4, which comprises preparing a compound of the formula I in which Y⁴ denotes -(CH₂)ₙ-COOH, Y³ is (C₁-C₄)-alkoxy and n is 2.

7. A process for the preparation of a peptide of the formula XI
P-NH-R³ (XI)
in which P represents a peptide residue composed of α-amino acids and R³ is as defined in claim 1, by solid-phase synthesis, which comprises coupling a compound of the formula I, using coupling reagents customary in peptide chemistry, via the -O-(CH₂)ₙ-COOH or -(CH₂)ₙ-COOH group to a resin, eliminating the protective group R², coupling on stepwise α-amino acids which are temporarily protected by amino protective groups which are base-labile or labile to weak acids, which amino acids are, where appropriate, in the form of their activated derivatives, and, after synthesis is complete, liberating the peptide of the formula XI from the resin by treatment with a moderately strong acid, with temporarily introduced side-chain protective groups being eliminated again simultaneously or by suitable measures subsequent thereto.

## Revendications (Revendications pour l'(les) Etat(s) contractant(s) suivant(s): AT, BE, CH, DE, FR, GB, GR, IT, LI, LU, NL, SE)

1. Composé de formule générale I 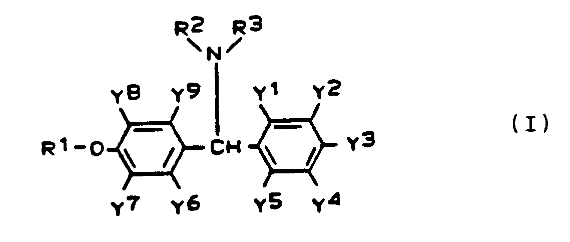 dans laquelle
R¹ représente un groupe alkyle en C₁-C₈,
R² représente un groupe protecteur de fonction amino séparable en milieu faiblement acide ou basique;
R³ représente un atome d'hydrogène ou un groupe alkyle en C₁-C₄;
Y¹, Y², Y³, Y⁴, Y⁵, Y⁶, Y⁷, Y⁸, Y⁹ représentent des atomes d'hydrogène ou des groupes alkyle en C₁-C₄, alcoxy en C₁-C₄ ou -O-(CH₂)ₙ-COOH, les radicaux pouvant être identiques ou différents, un radical étant toutefois -O-(CH₂)ₙ-COOH, ou
Y¹, Y², Y⁵, Y⁶, Y⁷, Y⁸, Y⁹ représentent un atome d'hydrogène ou un groupe alkyle en C₁-C₄ ou alcoxy en C₁-C₄, les radicaux pouvant être identiques ou différents, Y³ représente un groupe alcoxy en C₁-C₈ ou un atome d'hydrogène, et Y⁴ représente un groupe -(CH₂)ₙ-COOH ou -NH-CO-(CH₂)ₙ-COOH, et
n représente un nombre entier allant de 1 à 6.

2. Composé de formule I selon la revendication 1, dans lequel R¹ est le groupe méthyle et n est un nombre entier égal à 1, 2 ou 3.

3. Composé de formule I selon une ou plusieurs des revendications 1 et 2, dans lequel R² représente un groupe protecteur uréthanne et R³ représente un atome d'hydrogène.

4. Composé de formule I selon une ou plusieurs des revendications 1 à 3, dans lequel les radicaux Y¹-Y⁹ représentent le radical méthyle ou méthoxy, un radical étant toutefois -O-(CH₂)ₙ-COOH et au moins 4 de ces radicaux étant des atomes d'hydrogène, ou les radicaux Y¹, Y², Y⁵-Y⁹ représentent le radical méthyle ou méthoxy, mais au moins 4 de ces radicaux étant des atomes d'hydrogène, Y³ représente le groupe méthyle et Y⁴ représente le groupe -(CH₂)ₙ-COOH.

5. Composé de formule I selon une ou plusieurs des revendications 1 à 4, dans lequel un radical Y¹, Y³, Y⁵, Y⁷ ou Y⁸ représente -O-(CH₂)ₙ-COOH.

6. Composé de formule I selon une ou plusieurs des revendications 1 à 4, dans lequel Y⁴ représente -(CH₂)ₙ-COOH, Y³ représente un groupe alcoxy en C₁-C₄ et n = 2, ou Y⁴ représente -NH-CO-(CH₂)ₙ-COOH, Y¹ et Y³ représentent le groupe méthoxy et n = 2.

7. Procédé pour la préparation d'un composé de formule 1 selon une ou plusieurs des revendications 1 à 6, caractérisé en ce que
a) on fait réagir un composé de formule II 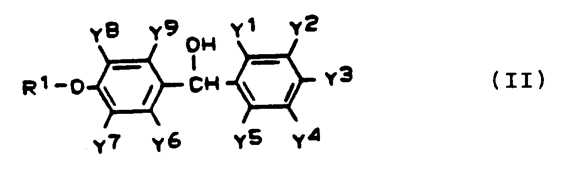 dans laquelle
R¹ représente un groupe alkyle en C₁-C₈, 2 3 4 5 6 7 8
Y¹, Y², Y³, Y⁴, Y⁵, Y⁶, Y⁷, Y⁸, Y⁹ représentent des atomes d'hydrogène ou des groupes alkyle en C₁-C₄, alcoxy en C₁-C₄ ou -O-(CH₂)ₙ-COOH, les radicaux pouvant être identiques ou différents, un radical étant toutefois -O-(CH₂)ₙ-COOH, ou
Y¹, Y², Y⁵, Y⁶, Y⁷, Y⁸, Y⁹ représentent un atome d'hydrogène ou un groupe alkyle en C₁-C₄ ou alcoxy en C₁-C₄, les radicaux pouvant être identiques ou différents, Y³ représente un groupe alcoxy en C₁-C₈ ou un atome d'hydrogène, et Y⁴ représente un groupe -(CH₂)ₙ-COOH ou -NH-CO-(CH₂)ₙ-COOH, et
n représente un nombre entier allant de 1 à 6,
avec un composé de formule III 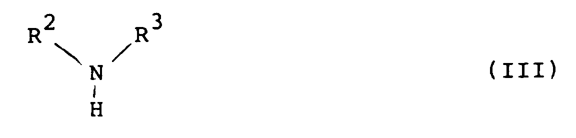 dans laquelle
R² représente un groupe protecteur de fonction amino séparable en milieu faiblement acide ou basique, et
R³ représente un atome d'hydrogène ou un groupe alkyle en C₁-C₄, ou
b) on fait réagir un composé de formule IV 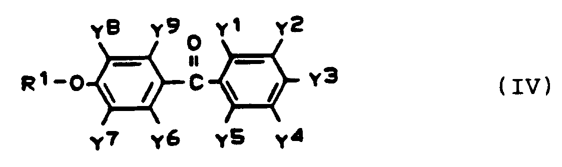 avec de l'hydroxylamine, pour aboutir à un composé de formule V 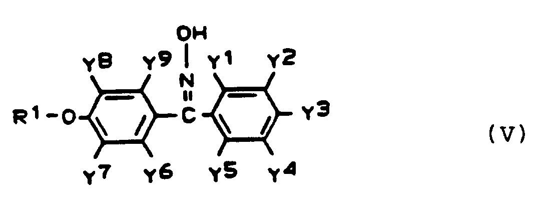 dans laquelle R¹, Y¹, Y², Y³, Y⁴, Y⁵, Y⁶, Y⁷, Y⁸ et Y⁹ sont tels que définis plus haut,
on réduit ensuite l'oxime en l'amine, on convertit éventuellement l'amine en composés de formule I dans lesquels R² représente un groupe protecteur de fonction amino, séparable en milieu faiblement acide ou basique, et R³ représente un atome d'hydrogène.

8. Utilisation d'un composé de formule I selon une ou plusieurs des revendications 1 à 6, dans la synthèse en phase solide de composés de formule XI
P―NH-R³ (XI)
dans laquelle P représente un reste peptidique constitué d'α-aminoacides et R³ est tel que défini dans la revendication 1.

9. Procédé pour la préparation d'un peptide de formule XI dans lequel P et R³ sont tels que définis dans la revendication 8, par synthèse en phase solide, caractérisé en ce que l'on fixe à une résine un composé de formule I, avec des réactifs de couplage usuels dans la chimie des peptides, par l'intermédiaire du groupement -O-(CH₂)ₙ-COOH, -(CH₂)ₙ-COOH ou -NH-CO-(CH₂)ₙ-COOH, on élimine le groupe protecteur R², on assemble graduellement des α-aminoacides éventuellement sous forme de leurs dérivés actives, et temporairement protégés par des groupes protecteurs de fonction amino labiles en présence de bases ou d'acides faibles, et une fois terminée la synthèse, on sépare d'avec la résine le peptide de formule XI par traitement par un acide de force moyenne, en séparant en même temps, ou à la suite de cela par des moyens appropriés, des groupes protecteurs de chaînes latérales, introduits temporairement.

## Revendications (Revendications pour l'(les) Etat(s) contractant(s) suivant(s): ES)

1. Procédé pour la préparation d'un composé de formule générale I 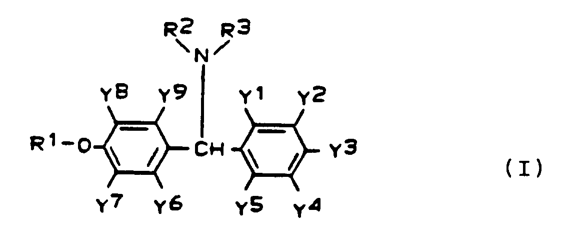 dans laquelle
R¹ représente un groupe alkyle en C₁-C₈,
R² représente un groupe protecteur de fonction amino séparable en milieu faiblement acide ou basique;
R³ représente un atome d'hydrogène ou un groupe alkyle en C₁-C₄;
Y¹, Y², Y³, Y⁴, Y⁵, Y⁶, Y⁷, Y⁸, Y⁹ représentent des atomes d'hydrogène ou des groupes alkyle en C₁-C₄, alcoxy en C₁-C₄ ou -O-(CH₂)ₙ-COOH, les radicaux pouvant être identiques ou différents, un radical étant toutefois -O-(CH₂)ₙ-COOH, ou
Y¹, Y², Y⁵, Y⁶, Y⁷, Y⁸, Y⁹ représentent un atome d'hydrogène ou un groupe alkyle en C₁-C₄ ou alcoxy en C₁-C₄, les radicaux pouvant être identiques ou différents, Y³ représente un groupe alcoxy en C₁-C₈ ou un atome d'hydrogène, et Y⁴ représente un groupe -(CH₂)ₙ-COOH ou -NH-CO-(CH₂)ₙ-COOH, et
n représente un nombre entier allant de 1 à 6,
caractérisé en ce que
a) on fait réagir un composé de formule II 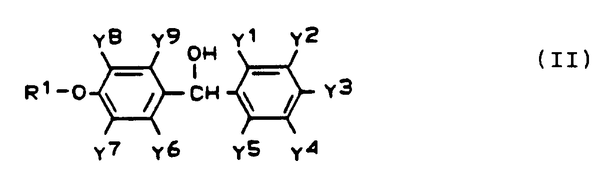 dans laquelle
R¹ représente un groupe alkyle en C₁-C₈,
Y¹, Y², Y³, Y⁴, Y⁵, Y⁶, Y⁷, Y⁸, Y⁹ représentent des atomes d'hydrogène ou des groupes alkyle en C₁-C₄, alcoxy en C₁-C₄ ou -O-(CH₂)ₙ-COOH, les radicaux pouvant être identiques ou différents, un radical étant toutefois -O-(CH₂)ₙ-COOH, ou
Y¹, Y², Y⁵, Y⁶, Y⁷, Y⁸, Y⁹ représentent un atome d'hydrogène ou un groupe alkyle en C₁-C₄ ou alcoxy en C₁-C₄, les radicaux pouvant être identiques ou différents, Y³ représente un groupe alcoxy en C₁-C₈ ou un atome d'hydrogène, et Y⁴ représente un groupe -(CH₂)ₙ-COOH ou -NH-CO-(CH₂)ₙ-COOH, et
n représente un nombre entier allant de 1 à 6,
avec un composé de formule III 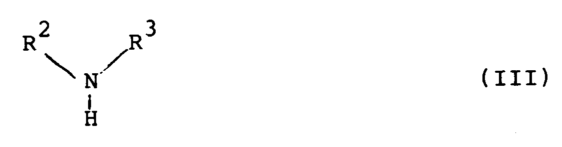 dans laquelle
R² représente un groupe protecteur de fonction amino séparable en milieu faiblement acide ou basique, et
R³ représente un atome d'hydrogène ou un groupe alkyle en C₁-C₄, ou
b) on fait réagir un composé de formule IV 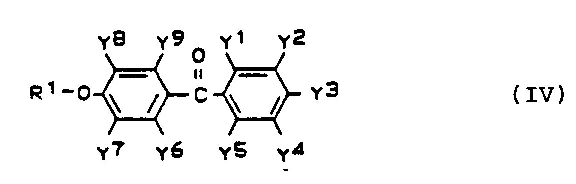 avec de l'hydroxylamine, pour aboutir à un composé de formule V 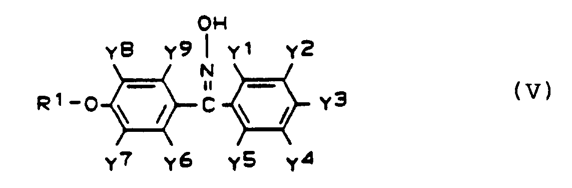 dans laquelle R¹, Y¹, Y², Y³, Y⁴, Y⁵, Y⁶, Y⁷, Y⁸ et Y⁹ sont tels que définis plus haut,
on réduit ensuite l'oxime en l'amine, on convertit éventuellement l'amine en composés de formule I dans lesquels R² représente un groupe protecteur de fonction amino, séparable en milieu faiblement acide ou basique, et R³ représente un atome d'hydrogène.

2. Procédé selon la revendication 1, caractérisé en ce que l'on prépare un composé de formule I dans lequel R¹ est le groupe méthyle et n est un nombre entier égal à 1, 2 ou 3.

3. Procédé selon une ou plusieurs des revendications 1 et 2, caractérisé en ce que l'on prépare un composé de formule I dans lequel R² représente un groupe protecteur uréthanne et R³ représente un atome d'hydrogène.

4. Procédé selon une ou plusieurs des revendications 1 et 3, caractérisé en ce que l'on prépare un composé de formule I dans lequel les radicaux Y¹-Y⁹ représentent le radical méthyle ou méthoxy, un radical étant toutefois -O-(CH₂)ₙ-COOH et au moins 4 de ces radicaux étant des atomes d'hydrogène, ou les radicaux Y¹, Y², Y⁵-Y⁹ représentent le radical méthyle ou méthoxy, mais au moins 4 de ces radicaux étant des atomes d'hydrogène, Y³ représentant le groupe méthyle et Y⁴ représentant le groupe -(CH₂)ₙ-COOH.

5. Procédé selon une ou plusieurs des revendications 1 à 4, caractérisé en ce que l'on prépare un composé de formule I dans lequel un radical Y¹, Y³, Y⁵, Y⁷ ou Y⁸ représente -O-(CH₂)ₙ-COOH.

6. Procédé selon une ou plusieurs des revendications 1 à 4, caractérisé en ce que l'on prépare un composé de formule I dans lequel Y⁴ représente -(CH₂)ₙ-COOH, Y³ représente un groupe alcoxy en C₁-C₄ et n = 2.

7. Procédé pour la préparation d'un peptide de formule XI
P―NH-R³ (XI)
dans laquelle P représente un reste peptidique constitué d'α-aminoacides et R³ est tel que défini dans la revendication 1, par synthèse en phase solide, caractérisé en ce que l'on fixe à une résine un composé de formule I, avec des réactifs de couplage usuels dans la chimie des peptides, par l'intermédiaire du groupement -O-(CH₂)ₙ-COOH ou -(CH₂)ₙ-COOH, on élimine le groupe protecteur R², on assemble graduellement des α-aminoacides éventuellement sous forme de leurs dérivés activés, et temporairement protégés par des groupes protecteurs de fonction amino labiles en présence de bases ou d'acides faibles, et une fois terminée la synthèse, on sépare d'avec la résine le peptide de formule XI par traitement par un acide de force moyenne, en séparant en même temps, ou à la suite de cela par des moyens appropriés, des groupes protecteurs de chaînes latérales, introduits temporairement.
